(19) Europäisches Patentamt
European Patent Office
Office européen des brevets

(11) **EP 3 623 970 A1**

(12) **EUROPEAN PATENT APPLICATION**
published in accordance with Art. 153(4) EPC

(43) Date of publication:
**18.03.2020 Bulletin 2020/12**

(51) Int Cl.:
***G06F 19/00*** *(2018.01)*

(21) Application number: **17908895.0**

(22) Date of filing: **15.12.2017**

(86) International application number:
**PCT/CN2017/116370**

(87) International publication number:
**WO 2018/205609 (15.11.2018 Gazette 2018/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **12.05.2017 CN 201710333821**

(71) Applicant: **BOE Technology Group Co., Ltd.
Beijing 100015 (CN)**

(72) Inventor: **ZHANG, Zhenzhong
Beijing 100176 (CN)**

(74) Representative: **Gesthuysen Patent- und
Rechtsanwälte
Patentanwälte
Huyssenallee 100
45128 Essen (DE)**

(54) **MEDICAL INTELLIGENT TRIAGE METHOD AND DEVICE**

(57) The present disclosure relates to the field of computer technology, particularly to an intelligent triage method and device as well as a computer readable storage medium. The intelligent triage method includes: extracting disease-related symptoms and signs from patient information as candidate factor information; obtaining a plurality of symptom-related candidate diseases and treatment measures from medical literature as identification knowledge information based on the candidate factor information ; matching the identification knowledge information with the candidate factor information; repeating the above steps until an affiliated department of a disease is determined or the patient information has been extracted and matched, and returning the affiliated department of the disease as a triage result. The method and the device analyze the interaction content with the patient to find out related knowledge and rules from the medical literature or other medical data so as to form medical evidences of the identification knowledge information, thereby analyzing the disease condition of the patient and giving relevant suggestions. This achieves the aim of matching a possible disease and the affiliated department thereof with main symptoms and signs of a patient and recommending an effective consultation department or consultation path.

```
┌─────────────────────────────────────┐
│  collecting patient information      │── S1
│  through a human-computer            │
│  interaction interface               │
└─────────────────────────────────────┘
                  │
                  ▼
┌─────────────────────────────────────┐
│  extracting disease-related          │── S2
│  symptoms and signs from the         │
│  patient information as candidate     │
│  factor information by a candidate    │
│  factor analyzer                      │
└─────────────────────────────────────┘
                  │
                  ▼
┌─────────────────────────────────────┐
│  obtaining a plurality of symptom-   │── S3
│  related candidate diseases and      │
│  treatment measures from medical     │
│  literature as identification         │
│  knowledge information based on the   │
│  candidate factor information by an   │
│  identification knowledge miner       │
└─────────────────────────────────────┘
                  │
                  ▼
┌─────────────────────────────────────┐
│  matching the identification          │── S4
│  knowledge information with the       │
│  candidate factor information by a    │
│  matcher                              │
└─────────────────────────────────────┘
                  │
                  ▼
┌─────────────────────────────────────┐
│  determining an affiliated            │── S5
│  department of a disease or having    │
│  extracted and matched the patient    │
│  information, and returning the       │
│  affiliated department of the         │
│  disease as a triage result           │
└─────────────────────────────────────┘
```

Fig. 1

**Description**

RELATED APPLICATION

The present application claims the priority of the Chinese patent application No. 201710333821.0 filed on May 12, 2017, which is entirely incorporated herein by reference.

TECHNICAL FIELD

The present disclosure relates to the field of computer technology, particularly to an intelligent triage method, an intelligent triage device and a computer readable storage medium.

BACKGROUND

When an outpatient department of a hospital performs triage, it is desperately desired by patients to accurately and quickly determine departments required by the patients. However, the outpatient triage scene in reality is that the patients learn little about the department classification of the hospital, they can only seek advice briefly from the staff at the registration window or the triage station which department(s) they should register, when registering. However, specialist only masters his own field, most of the staff at the registration window or the triage station are not professional doctors. Hence, the result confronted by the patients is always that after they have registered after all the troubles, they were informed by the doctors that the disease does not conform to the registered department, and they have to register another department for symptomatic treatment. At this time, the patients either register again or ask the doctors to prescribe a drug that is probably symptomatic. However, there are only limited registration tickets of a doctor. If a patient registers again, he can only see the doctor another day, such that the golden period for diagnosis of the disease will be missed. For rarer specialist registration tickets, the problem that the disease does not conform to the registered department or the doctor can only prescribe a reservation for examination a few days later also exists. In addition to delay of diagnosis of the disease, these situations often accompany with high time costs and traffic costs.
The above situations are still far from the scientific triage. The current triage devices are generally based on the manually programmed rule library, which has to take much manpower and time, and has a narrow application scope. With the rise of artificial intelligence and the popularization of humanistic care, the intelligent triage starts to develop. Compared to the conventional outpatient triage, the intelligent triage can determine the diseases more quickly and accurately and give reasonable suggestions. For example, a proposed triage method is a triage mode of Airdoc based on deep learning method. It is generally an end-to-end application to use the deep learning method, the process of which is unexplainable for people, and lacks good information interaction. This is a great disadvantage for medical application.

SUMMARY

In order to solve or mitigate at least one defect in the above prior art, the present disclosure provides an intelligent triage method, an intelligent triage device and a computer readable storage medium. They can mine related medical knowledge from medical literature through information provided by a patient based on knowledge mining and semantic relation, and select interaction content with the patient automatically, so as to determine a triage condition of the patient more quickly and accurately.
According to an aspect of the present disclosure, an intelligent triage method is provided. The method can comprise: extracting disease-related symptoms and signs from patient information as candidate factor information by a candidate factor analyzer; obtaining a plurality of symptom-related candidate diseases and treatment measures from medical literature as identification knowledge information based on the candidate factor information by an identification knowledge miner; matching the identification knowledge information with the candidate factor information by a matcher; repeating the above steps until an affiliated department of a disease is determined or the patient information has been extracted and matched, and returning the affiliated department of the disease as a triage result.
In an embodiment, the patient information can be obtained from oral expressions or electronic inputs of the patient through a human-computer interaction interface. Returning the determined department as the triage result can comprise broadcasting the determined department in voice through the human-computer interaction interface or displaying electronic information text of the determined department.
In an embodiment, the candidate factor information can include key words or key phrases and time events of the disease-related symptoms and sign. The step of extracting the candidate factor information can comprise: extracting, by the candidate factor analyzer, the key words or key phrases and time events from oral expressions provided by a patient through natural language processing technology or from information text provided by a patient through information extracting technology.

In an embodiment, the step of obtaining the identification knowledge information can comprise: retrieving key words or key phrases related document contents from medical literature based on the key words or the key phrases in the candidate factor information by a content retriever; finding and mining disease-related knowledge from the retrieved document contents using natural language processing technology by a knowledge extractor.

In an embodiment, the step of retrieving key words or key phrases related document contents from medical literature can comprise: performing participle and named-entity identification to the medical literature using the natural language processing technology and setting up inverted indexes by the content retriever; and retrieving a document containing all the key words or key phrases from the inverted indexes based on the key words or the key phrases in the candidate factor information by the content retriever.

In an embodiment, the step of finding and mining disease-related knowledge from the retrieved document contents using the natural language processing technology can comprise: determining segments in the document where the key words or the key phrases locate, and extracting symptom and sign related candidate diseases through sematic relation by the knowledge extractor.

In an embodiment, the step of matching the identification knowledge information with the candidate factor information can comprise: if only one of the candidate diseases is outputted, determining the disease and a corresponding affiliated department based on the candidate factor; if a plurality of the candidate diseases are outputted, determining the affiliated department based on prevalence of diseases with the same symptoms and signs in a specified period of time or a specified region; or, further selecting information having the maximum discrimination between different diseases with crossed symptoms and signs as an extension question, to further obtain patient information and extract new candidate factor information so as to further perform matching.

In an embodiment, the discrimination between different diseases with crossed symptoms and signs can be determined through information gains. The larger an information gain is, the larger the discrimination will be. The smaller an information gain is, the smaller the discrimination will be. A calculation formula of an information gain can be:

$$IG(Symptom) = H(Disease) - H(Disease|Symptom)$$

wherein, Symptom represent a symptom, Disease represents a disease, H(.) represents an entropy.

According to another aspect of the present disclosure, an intelligent triage device is provided, comprising a candidate factor analyzer, an identification knowledge miner and a matcher. The candidate factor analyzer can be configured to extract disease-related symptoms and signs from patient information as candidate factor information. The identification knowledge miner is connected with the candidate factor analyzer, and can be configured to obtain a plurality of symptom-related candidate diseases and treatment measures from medical literature as identification knowledge information based on the candidate factor information. The matcher is connected with the identification knowledge miner, and can be configured to match the identification knowledge information with the candidate factor information so as to determine an affiliated department of a disease, and return the determined department as a triage result.

In an embodiment, the device can further comprise a human-computer interaction facility. The human-computer interaction facility can be connected with the candidate factor analyzer and the matcher respectively. The human-computer interaction facility provides a human-computer interaction interface for a patient, and can be configured to collect patient information and display a triage result to a patient. The patient information can be obtained from oral expressions or electronic inputs of the patient. The triage result can be returned to the patient through voice broadcasting or electronic information text display.

In an embodiment, the candidate factor information can include key words or key phrases and time events of the disease-related symptoms and signs. The candidate factor analyzer can be configured to extract the key words or key phrases and time events from oral expressions provided by a patient through natural language processing technology or from information text provided by a patient through information extracting technology.

In an embodiment, the identification knowledge miner can comprise a content retriever and a knowledge extractor. The content retriever can be configured to retrieve key words or key phrases related document contents from medical literature based on the key words or the key phrases outputted by the candidate factor analyzer. The knowledge extractor is configured to find and mine disease-related knowledge from the retrieved document contents using the natural language processing technology.

In an embodiment, the content retriever can be configured to: perform participle and named-entity identification to medical literature using natural language processing technology and set up inverted indexes; and retrieve a document containing all the key words or key phrases from the inverted indexes based on the key words or the key phrases outputted by the candidate factor analyzer.

In an embodiment, the knowledge extractor can be configured to determine segments in the document where the key words or the key phrases locate, and extract symptom and sign related candidate diseases through sematic relation.

In an embodiment, if only one of the candidate diseases is outputted, the matcher can be configured to determine the disease and a corresponding affiliated department based on the candidate factor. If a plurality of the candidate diseases are outputted, the matcher can be configured to determine the affiliated department based on prevalence of diseases with the same symptoms and signs in a specified period of time or a specified region; or, the matcher can be configured to further select information having the maximum discrimination between different diseases with crossed symptoms and signs as an extension question, to further obtain patient information and extract new candidate factor information so as to further perform matching.

In an embodiment, the matcher can be configured to determine discrimination between different diseases with crossed symptoms and signs through information gains. The larger an information gain is, the larger the discrimination will be. The smaller an information gain is, the smaller the discrimination will be. A calculation formula of an information gain can be:

$$IG(Symptom) = H(Disease) - H(Disease|Symptom)$$

wherein, Symptom represent a symptom, Disease represents a disease, H(.) represents an entropy.

According to another aspect of the present disclosure, an intelligent triage device is provided, comprising: one or more processors and a memory. The memory stores computer executable instructions thereon. The computer executable instructions are configured to, when executed by the one or more processors, carry out any one of the above-described methods.

According to another aspect of the present disclosure, a computer readable storage medium is provided, containing computer executable instructions thereon. The instructions, when executed by one or more processors, enable the one or more processors to carry out any one of the above-described methods.

Some embodiments of the present disclosure can achieve at least one of the following beneficial effects and/or other beneficial effects: the intelligent triage method and the intelligent triage device analyze interaction content with a patient to find out related knowledge and rules from medical literature or other medical data so as to form medical evidences of identification knowledge information, thereby analyzing a disease condition of the patient and giving relevant suggestions. This achieves the aim of matching the possible disease and the affiliated department thereof with the main symptoms and signs of the patient and recommending an effective consultation department or consultation path.

The SUMMARY introduces some concepts of the present invention in a simplified form. These concepts will be further described in the detailed description. The SUMMARY is not intended to provide essential features or substantive features of the claimed subject matter, nor to limit the scope of the claimed subject matter. In addition, just as described herein, various other features and advantages can also be combined into these technologies as needed.

BRIEF DESCRIPTION OF THE DRAWINGS

In order to explain the technical solutions of some embodiments of the present disclosure more clearly, the present disclosure provides the following drawings for use in describing the embodiments. It should be aware that the drawings in the following description only involve some embodiments. For the ordinary skilled person in the art, other drawings can also be obtained from these drawings without undue experimentation, and also fall within the scope of the present invention.

Fig. 1 is a flow chart of an intelligent triage method according to an embodiment;

Fig. 2 is a schematic view of architecture of an intelligent triage device according to an embodiment;

Fig. 3 is a schematic view of a triage example according to an embodiment.

Reference signs:

1. human-computer interaction facility;

2. candidate factor analyzer;

3. identification knowledge miner;

4. matcher;

5. patient;

6. medical literature.

DETAILED DESCRIPTION

In order to enable the skilled person in the art to understand the technical solutions of the present disclosure better, the intelligent triage method, the intelligent triage device and the computer readable medium of the present disclosure will be further described below in detail in conjunction with the drawings and the embodiments.

The technical concept of some embodiments of the present invention lies in: the intelligent triage means determining possible diseases based on the main symptoms and signs of patients, determining order of importance and emergency of the diseases and the affiliated department thereof, and recommending effective consultation paths. The intelligent triage methods and the corresponding intelligent triage devices according to some embodiments mine related medical knowledge from the medical literature through information provided by the patient based on knowledge mining and semantic relation, and automatically select interaction content related with the patient and the disease, so as to determine triage of the patient more quickly and accurately.

With respect to the problem that the current triage, in addition to delay of disease diagnosis, often accompanies with high time costs and traffic costs, the above intelligent triage method realizes automatic selection of interaction content with the patient by performing triage based on knowledge mining, so as to determine the triage department of the patient more quickly and accurately.

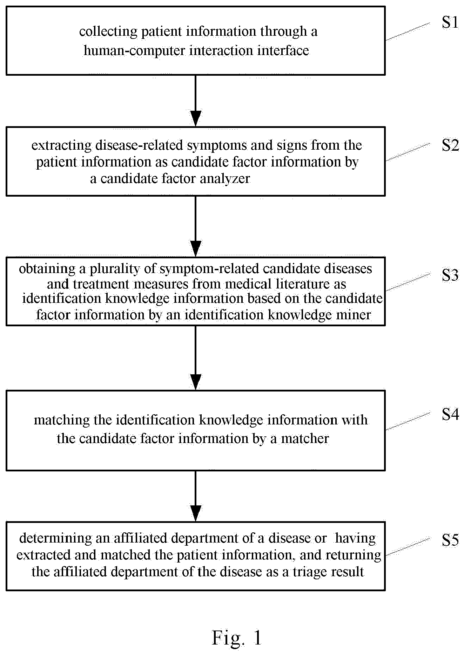

Fig. 1 shows a flow chart of an intelligent triage method according to an embodiment. As shown in Fig. 1, the intelligent triage method can comprise steps S1-S5.

Step S1) collects patient information through a human-computer interaction interface.

At this step, the patient information can be obtained from oral expressions or electronic inputs of patient through the human-computer interaction interface. The triage results can also be returned to the patients through voice broadcast or electronic information text display using the human-computer interaction interface. Specifically, the patient information can be collected in various ways, for example, human-computer text interaction or voice interaction, so as to obtain as much as possible information of patients including main symptoms.

It should be understood herein that the patient information can be obtained all at once for subsequent analysis and matching processing, and can also be obtained step by step, so as to gradually provide more new patient information for analysis and matching processing.

Step S2) extracts disease-related symptoms and signs from the patient information as candidate factor information by a candidate factor analyzer.

At this step, the candidate factor analyzer analyzes the information provided by the patient and extracts therefrom disease-related main symptoms and main signs as the candidate factor information. In an embodiment, time events and key words or key phrases for the disease-related symptoms and signs can be extracted from the oral expressions provided by the patient through natural language processing technology or from information text provided by the patient through information extracting technology. The key words or key phrases and the time events can serve as the candidate factor information.

Step S3) obtains a plurality of symptom-related candidate diseases and treatment measures from medical literature as identification knowledge information based on the candidate factor information by an identification knowledge miner.

At this step, the identification knowledge miner can mine related knowledge information of main symptom related diseases and treatment measures from massive medical literature. That is, key words or key phrases related document contents can be retrieved from the medical literatures based on the key words or key phrases in the candidate factor information, and disease-related knowledge can be found and mined from the retrieved document contents using the natural language processing technology.

The step of obtaining related identification knowledge information such as main symptom related diseases and treatment measures from the medical literature can further comprise:

Step S31): A content retriever retrieves related contents from the medical literature based on the key words or key phrases outputted at step S2). That is, the content retriever performs participle and named-entity identification to medical literature using natural language processing technology, and sets up inverted indexes; and retrieves a document containing all the key words or key phrases from the inverted indexes based on the key words or key phrases outputted at step S2).

Step S32): A knowledge extractor finds and mines disease-related knowledge from the retrieved document using the natural language processing technology. That is, the knowledge extractor determines segments in the document where the key words or the key phrases locate, and extracts main symptom and main sign related candidate diseases through sematic relation.

Step S4) matches the identification knowledge information with the candidate factor information by a matcher.

At this step, the matcher extracts medical evidences from the related knowledge information of the main symptom related diseases and treatment measures obtained at step S3), and performs matching to the candidate factor information based on the medical evidences. The matching may include the following:

if only one candidate disease is outputted, determining the candidate disease and a corresponding affiliated department based on the candidate factor;

if a plurality of candidate diseases are outputted, determining the affiliated department based on prevalence of diseases with the same symptoms and signs in a specified period of time or a specified region; or, further selecting information having maximum discrimination between different diseases with crossed symptoms and signs to use as an extension question, to further obtain patient information and extract new candidate factor information, so as to further perform matching. The way of obtaining the patient information herein can be further asking the patient so as to further obtain the disease-related information, and determine a triage result based on the disease-related candidate factor and information of possible diseases, so as to obtain the triage result.

In an embodiment, the discrimination between different diseases with crossed symptoms and signs can be determined through information gain. The larger the information gain is, the larger the discrimination will be. The smaller the information gain is, the smaller the discrimination will be. The calculation formula of the information gain can be:

$$IG(Symptom) = H(Disease) - H(Disease|Symptom)$$

wherein Symptom represents the symptom, Disease represents the disease, and H(.) represents an entropy.
The actually most possible triage result can be obtained by further screening and positioning a plurality of candidate diseases.
Step S5) repeats the above steps until an affiliated department of the disease is determined or the patient information has been extracted and matched, and returning the affiliated department of the disease as a triage result.
The steps S1)-S4) are repeated until the affiliated department of the disease is determined, or no disease-related information can be obtained from the patient information any more. Then the affiliated department of the disease is returned and the triage result of the possible disease is displayed to the patient.
Reciprocated interaction and analysis are performed by means of the above steps until the triage result is returned automatically. The triage result starts from the patient information to extract disease-related symptoms and signs as the candidate factor information, and automatically finds and mines disease-related identification knowledge information from massive medical literature. Hence, the triage result is closest to the department that should be registered actually.
According to another aspect of the present disclosure, an intelligent triage device is further provided. The triage is also performed based on knowledge mining. The triage device can automatically select disease-related candidate factor information in the patient information, so as to determine the triage department of the patient more quickly and accurately.

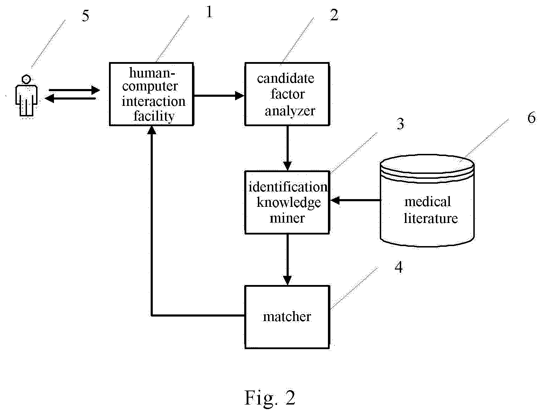

Fig. 2 shows a schematic view of architecture of an intelligent triage device according to an embodiment. As shown in Fig. 2, the intelligent triage device can comprise a human-computer interaction facility 1, a candidate factor analyzer 2, an identification knowledge miner 3 and a matcher 4.
The human-computer interaction facility 1 can provide a human-computer interaction interface for a patient 5, and can be configured to collect patient information and display a triage result to the patient 5. The patient information can be obtained from oral expressions or electronic inputs of the patient. The triage result can be returned to the patient through voice broadcast or electronic information text display.
The candidate factor analyzer 2 is connected with the human-computer interaction facility 1, and can be configured to extract disease-related symptoms and signs from the patient information as the candidate factor information. For example, the candidate factor analyzer 2 can be configured to analyze information provided by the patient 5 and collected by the human-computer interaction facility 1, and extract therefrom disease-related main symptoms and main signs as the candidate factor information. In an embodiment, the candidate factor analyzer 2 can be configured to extract key words or key phrases and time events for the disease-related symptoms and signs from the oral expressions provided by the patient through the natural language processing technology or from the information text provided by the patient through the information extracting technology. The key words or key phrases and time events can serve as the candidate factor information. The disease candidate factors can be obtained by extracting the disease-related information in the patient information by the candidate factor analyzer 2.

The identification knowledge miner 3 is connected with the candidate factor analyzer 2, and can be configured to obtain a plurality of symptom-related candidate diseases and treatment measures from medical literature as identification knowledge information based on the candidate factor information. The related knowledge information of the main symptom related diseases and treatment measures can be mined from massive medical literature 6 based on the information extracted from the candidate factor analyzer 2, so as to obtain information of possible diseases corresponding to the disease candidate factors.

In an embodiment, the identification knowledge miner 3 can comprise a content retriever and a knowledge extractor. The content retriever can be configured to retrieve key words or key phrases related document contents from the medical literature based on the key words or the key phrases outputted by the candidate factor analyzer. The knowledge extractor can be configured to find and mine disease-related knowledge from the retrieved document contents using the natural language processing technology.

The content retriever can be configured to perform participle and named-entity identification to the medical literature using the natural language processing technology and set up inverted indexes; and retrieve a document containing all the key words or key phrases from the inverted indexes based on the key words or the key phrases outputted by the candidate factor analyzer. The knowledge extractor can be configured to determine segments in the document where the key words or the key phrases locate, and extract symptom and sign related candidate diseases through sematic relation.

The matcher 4 can be connected with the human-computer interaction facility 1 and the identification knowledge miner 3 respectively, and can be configured to match the identification knowledge information with the candidate factor information, so as to return a triage result. The triage result of the possible disease of the patient 5 can be matched by comparing the knowledge outputted from the identification knowledge miner 3 with the candidate factor information. If only one candidate disease is outputted, the matcher 4 can be configured to determine the disease and a corresponding affiliated department based on the candidate factor. If a plurality of candidate diseases are outputted, the matcher 4 can be configured to determine the affiliated department based on prevalence of diseases with the same symptoms and signs in a specified period of time or a specified region; or, further select information having maximum discrimination between different diseases with crossed symptoms and signs to use as an extension question, to further obtain patient information and extract new candidate factor information so as to further perform matching.

The matcher 4 can determine discrimination between different diseases with crossed symptoms and signs through information gain. The larger the information gain is, the larger the discrimination will be. The smaller the information gain is, the smaller the discrimination will be. The calculation formula of the information gain can be:

$$IG(Symptom) = H(Disease) - H(Disease|Symptom)$$

wherein Symptom represents the symptom, Disease represents the disease, and H(.) represents an entropy.

The actually most possible triage result can be obtained by further screening and positioning a plurality of candidate diseases by the matcher 4.

According to another aspect of the present disclosure, the intelligent triage device can also be implemented by one or more processors and a memory. The memory stores computer executable instructions thereon. The computer executable instructions are configured to, when executed by the one or more processors, carry out any method as stated above.

According to another aspect of the present disclosure, a computer readable storage medium is provided, comprising computer executable instructions thereon. The instructions, when executed by one or more processors, enable the one or more processors to carry out any method as stated above.

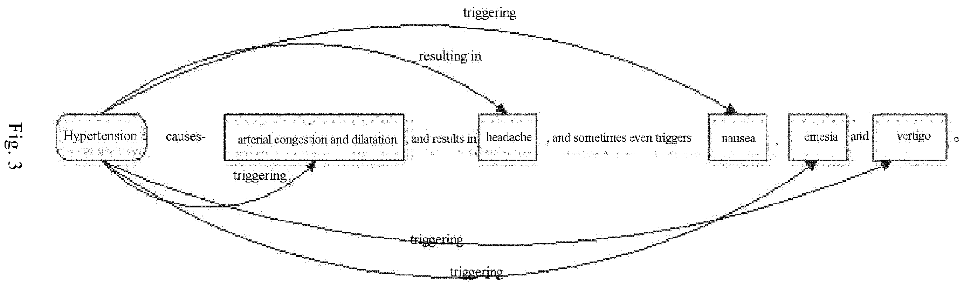

Fig. 3 shows a schematic view of a triage example according to an embodiment. Functions and implementations of modules of the above intelligent triage method and the intelligent triage device will be explained below in detail in conjunction with a triage example by referring to Fig. 3.

It shall be noted that corresponding relation between the four modules in the intelligent triage device and the five steps in the intelligent triage method is: the human-computer interaction facility 1 involves steps S1) and step S5), the candidate factor analyzer 2 involves step S2), the identification knowledge miner 3 involves step S3) and the matcher 4 involves step S4).

Firstly, the human-computer interaction facility 1 provides a human-computer interaction interface for the patient 5, and can be configured to collect information such as symptoms and signs provided by the patient 5. For example, the patient information can be formed by asking the patient 5 about the uncomfortable parts or the corresponding symptoms, signs etc. and then collecting the reply information of the patient 5. The human-computer interaction facility 1 can be further configured to display the final triage result of the intelligent triage device to the patient 5.

Subsequently, the candidate factor analyzer 2 analyzes the patient information collected by the human-computer interaction facility 1, and extract therefrom main information such as disease-related symptoms and signs. In an embodiment, key word or key phrases (the key words or key phrases herein refer to disease-related symptoms and signs etc.) and time events can be extracted from voice or text of the patient information through the natural language processing technology and the information extracting technology. For example, the device asks "what's wrong with your?", the patient 5 may answer "the stomachache began yesterday, and the backache also begins today". From this example, the candidate factor analyzer 2 can extract a key word "stomachache" and a key phrase "the backache also begins" automatically. Meanwhile, the temporal nouns "yesterday" and "today" can be extracted, so as to obtain time and event (yesterday, stomachache) and (today, stomachache and backache) from analysis.

Afterwards, the identification knowledge miner 3 mines related knowledge information from massive medical literature 6 based on the information extracted by the candidate factor analyzer 2. From the preceding content it can be seen that: the identification knowledge miner 3 can comprise a content retriever and a knowledge extractor.

The content retriever can retrieve related content from the medical literature 6 based on the key words or key phrases outputted by the candidate factor analyzer 2. In an embodiment, it can comprise the following process: firstly performing participle and named-entity identification to the medical literature 6 using the natural language processing technology, and setting up inverted indexes which can take the following form: "word 1 "-"document 1, document i, ..., document N", wherein "document 1, document i, and document N" are all documents containing the "word 1"; and then retrieving documents containing all of the key words or key phrases from the inverted indexes based on the key words or key phrases outputted by the candidate factor analyzer 2. "wl, w2, ..., wK" are K key words or key phrases, and "S1, S2, ..., SK" are corresponding document sets in the inverted indexes (i.e., S1 is a document set containing wl, and so forth). Thus the retrieved document sets are S={S1∩S2∩...∩SK}. For example, assuming that the key words are "headache" and "vertigo", a document set corresponding to "headache" in the inverted indexes is {"document 1", "document 2", "document 3"}, and a document set corresponding to "vertigo" is {"document 2", "document 4", "document 6"}, the retrieved eligible document set is {"document 2"}.

The knowledge extractor can mine related knowledge from the retrieved document using the natural language processing technology. In an embodiment, it can comprise the following process: determining segments in the document where the key words or key phrases locate, then extracting symptom and sign (key words or key phrases, such as headache, vertigo etc.) related candidate diseases through semantic relation. For example, given the key words "headache" and "vertigo", a segment "the hypertension may cause arterial congestion and dilatation, and result in headache, and sometimes even trigger nausea, emesia and vertigo." is determined from the received document. Through semantic relation extraction, the knowledge extractor can obtain the following semantic relation: the hypertension triggers headache, the hypertension triggers nausea, the hypertension triggers emesia, and the hypertension triggers vertigo, and so on.

Through the above semantic relation, the content retriever mines knowledge triggering (hypertension, headache), triggering (hypertension, nausea), triggering (hypertension, emesia), triggering (hypertension, vertigo)}, thereby taking "hypertension" as the candidate disease that causes headache and vertigo of the patient 5.

Finally, the triage of the possible disease of the patient 5 is analyzed by the matcher 4 from the knowledge outputted by the identification knowledge miner 3 and the candidate factor information. In an embodiment, the analysis can comprise the following process:

If only one candidate disease is outputted by the identification knowledge miner 3, the disease and the corresponding department will be outputted. For example, if a candidate disease outputted by the identification knowledge miner 3 is only "hypertension" with respect to "headache" and "vertigo", "hypertension" and "Internal Medicine-Cardiovascular Department" that shall be registered will be outputted; and

If a plurality of candidate diseases is outputted, information having the maximum discrimination can be chosen as a question to consult to the patient 5. Specifically, the discrimination is determined through information gain. The larger the information gain is, the larger the discrimination will be. The smaller the information gain is, the smaller the discrimination will be. For example, with respect to the above example, if the outputted candidate diseases include "hypertension", "migraine" and "panasthenia", the returned knowledge is as shown in Table 1:

Table 1 Returned Knowledge Table

| Disease | relation | Symptoms or signs |
|---------|----------|-------------------|
| hypertension | trigger | Headache, vertigo, nausea, diuresis |

(continued)

| Disease | relation | Symptoms or signs |
|---|---|---|
| migraine | trigger | Headache, vertigo, nausea, emesia |
| panasthenia | trigger | Headache, vertigo, insomnia, anxiety, irritable |

From Table 1 it can be seen that a possible disease of the patient 5 cannot be determined only from "headache" and "vertigo". The disease might be any one of hypertension, migraine and panasthenia. Hence, in order to perform further interactive quiz based on the new knowledge, the device needs to communicate with the patient 5 through the human-computer interaction facility 1, to collect new patient information so as to determine possible disease symptoms or signs. With respect to the above example, the device can ask a question "Do you have any symptoms of nausea?" or "Have you been suffering from insomnia?" or any other questions of the related symptoms. Since there are generally a great many diseases and symptoms, it becomes very important how the device select the symptoms so as to determine disease information of the patient 5 as quickly as possible.

The actually most possible triage result is obtained by further screening and positioning information of the plurality of candidate diseases. The intelligent triage method and the intelligent triage device select the symptoms based on two aspects:

1) Prevalence of diseases: i.e., proportions occupied by the diseases of the patients with the same symptoms that have been confirmed by the hospital. For example, assuming that in 100 persons with the symptoms of "headache and vertigo", 70 persons are confirmed as hypertension, 20 persons are confirmed as migraine, and 10 persons are confirmed as panasthenia, the prevalence of hypertension is popular (hypertension) = 70%, popular(migraine) = 20%, and popular (panasthenia) = 10%. The intelligent triage method of this embodiment takes the prevalence of the diseases as priori probability. In the event that other conditions are the same, symptoms of a disease with the largest prevalence is selected preferentially. For example, under the above proportions of prevalence of the diseases, the matched possible disease in Table 1 is hypertension.

2) Discrimination of symptoms: i.e., whether symptoms can distinguish diseases. For example, with respect to the above example, if the device selects to ask "Do you have a symptom of nausea?" and the patient 5 answers "yes", the device still cannot determine whether the patient 5 has hypertension or migraine because these two diseases can both cause the symptom of nausea. Thus it may also need to further obtain new candidate factors.

The intelligent triage method of this embodiment uses the information gain formula to calculate discrimination of symptoms, i.e.,

$$IG(Symptom) = H(Disease) - H(Disease|Symptom)$$

wherein Symptom represents the symptom, Disease represents the disease, and H(.) represents an entropy.

Based on information in Table 1, assume that a patient with symptoms of "headache and vertigo" comes for triage, at this time, the device cannot determine whether the patient should be triaged to "hypertension", "migraine" or "panasthenia" because these three diseases might all cause the above symptoms. In order to further determine the patient's condition, the device needs to collect more patient information.

The device firstly determines based on prevalence of diseases which one of the diseases with the above symptoms has the most patients. For example, in this example, assume that the proportion occupied by the patients of hypertension is 70% (this percentage can be obtained from statistics of medical records confirmed by the hospital), i.e., in most cases, a patient with symptoms of "headache and vertigo" has hypertension. At this time, the device will choose one symptom from hypertension related symptoms obtained by the identification knowledge miner 3, to ask the patient so as to determine whether the patient has hypertension.

Secondly, in addition to "headache and vertigo", the hypertension has two related symptoms of "nausea" and "diuresis". For these two symptoms, the device calculates their information gains (IGs) respectively and chooses the symptom with the larger information gain to obtain related information from the patient. Assume that "hypertension", "migraine" and "panasthenia" conform to uniform distribution. then, p(hypertension) = p(migraine" = p(panasthenia) = 1/3, wherein p(.) represents probability. At this time, an entropy of diseases related to the symptoms of "headache and vertigo" is:

$$H(Disease) = -\frac{1}{3}\log_2^{\frac{1}{3}} - \frac{1}{3}\log_2^{\frac{1}{3}} - \frac{1}{3}\log_2^{\frac{1}{3}} = \log_2^3$$

Because diseases with the symptom of "nausea" in this example only include hypertension and migraine, when the symptom is "nausea", p(hypertension) = 1/2, p(migraine) = 1/2, p(panasthenia) = 0. At this time, an entropy of diseases related to the symptom of "nausea" is:

$$H(Disease|nausea) = -\frac{1}{2}log_2^{\frac{1}{2}} - \frac{1}{2}log_2^{\frac{1}{2}} = log_2^2.$$

Then, the information gain formula according to the discrimination of symptoms is:

$$IG(nausea) = log_2^3 - log_2^2 = log_2^{1.5}$$

Because diseases with the symptom of "diuresis" in this example only incude hypertension, when the symptom is "diuresis", p(hypertension) = 1, p(migraine) = 0, p(panasthenia) = 0. At this time, an entropy of diseases related to the symptom of "diuresis" is:

$$H(Disease|diuresis) = -1 \times log_2^1 = 0$$

$$IG(diuresis) = log_2^3 - 0 = log_2^3$$

Because IG(diuresis)>IG(nausea), the device chooses the symptom of "diuresis" to further obtain information or ask a question (e.g., Do you have diuresis recently?). If the information or answer provided by the patient is "yes", it is determined as a triage result of hypertension. If the answer is "no", the triage result of hypertension will be excluded, and the rest of the diseases will be taken as the candidate diseases. It is shown that for this example, the triage matching is namely repeating the above steps to "migraine" and "panasthenia" until the disease triage is determined or the patient terminates this process.

To sum up, the intelligent triage method and the corresponding intelligent triage device of this embodiment firstly select a disease with the maximum prevalence based on the prevalence of diseases, and then select from the disease a symptom having the maximum discrimination as a question to ask the patient 5 through the human-computer interaction facility 1. For example, for the above example, firstly the hypertension is selected as the most possible disease of the patient 5 (because the prevalence of the hypertension is the maximum, which is 70%), and then other symptoms of the hypertension are found from knowledge outputted by the knowledge extractor. A symptom of "diuresis" which has the maximum discrimination is selected to produce a question of "Do you have diuresis recently?" to ask the patient 5 through the human-computer interaction facility 1. Repetition is made in this way until a disease triage is determined or no new disease candidate factor information can be obtained any more. At this time, a triage result matched by the device is displayed to the user through the human-computer interaction facility 1.

The intelligent triage method and the intelligent triage device analyze interaction content with a patient to find out related knowledge and rules from medical literature or other medical data so as to form medical evidences of identification knowledge information, thereby analyzing diseases of the patient and giving relevant suggestions. This achieves the aim of matching a possible disease and an affiliated department thereof with main symptoms and signs of a patient, and recommending an effective consultation department or consultation path.

The above knowledge mining based intelligent triage method and intelligent triage device have two advantages as follows:

1) Interaction content with a patient can be selected automatically by analyzing information provided by the patient and related medical knowledge, so as to determine a condition of an affiliated department of a disease of the patient more quickly and accurately;

2) Related evidences are determined through knowledge mining. These evidences are described using the natural language and are explainable. Then, all possible conditions of the patient are listed out based on support degrees, and reasonable suggestions are given.

It can be understood that the above implementations are merely exemplary implementations used for explaining the principle of the present invention. However, the present invention is not limited to this. It should be noted that without departing from the spirit and the essence of the present invention, various variations and improvements can be made for the ordinary skilled person in the art. These variations and improvements are also encompassed within the protected scope of the present invention. The protected scope of the present invention should be subject to the protected scope of the attached claims.

It shall be noted that the above embodiments only make exemplary illustrations based on division of the above functional modules. In actual applications, the above functions can be allocated to different functional modules for implementing as needed. Internal structure of a device can be divided into different functional modules so as to implement all or part of the functions described above. Also, the function of one of the above modules can be implemented by a plurality of modules, and the functions of multiple of the above modules can also be integrated into one module for implementing.

In the Claims, none of references signs placed in parentheses should be interpreted as limitations to the claims. The term "comprising" does not exclude presence of elements or steps other than the elements or steps listed in the claims. The word "a" or "an" prior to an element does not exclude presence of a plurality of such an element. The present invention can be carried out by means of hardware comprising several separate elements, appropriately programmed software or firmware, or any combination thereof.

In device or system claims in which several apparatuses are listed, one or more of these apparatuses can be embodied in a same hardware item. The fact that only certain measures are recited in mutually different dependent claims does not indicate that combination of these measures cannot be used advantageously.

## Claims

1. An intelligent triage method comprising:

   extracting disease-related symptoms and signs from patient information as candidate factor information by a candidate factor analyzer;
   obtaining a plurality of symptom-related candidate diseases and treatment measures from medical literature as identification knowledge information based on the candidate factor information by an identification knowledge miner;
   matching the identification knowledge information with the candidate factor information by a matcher;
   repeating the above steps until an affiliated department of a disease is determined or the patient information has been extracted and matched, and returning the affiliated department of the disease as a triage result.

2. The intelligent triage method according to claim 1, wherein the patient information is obtained from oral expressions or electronic inputs of a patient through a human-computer interaction interface, and returning the determined department as the triage result comprises broadcasting the determined department in voice through the human-computer interaction interface or displaying electronic information text of the determined department.

3. The intelligent triage method according to claim 1, wherein the candidate factor information includes key words or key phrases and time events of the disease-related symptoms and signs, and the step of extracting the candidate factor information comprises: extracting, by the candidate factor analyzer, the key words or key phrases and the time events from oral expressions provided by a patient through a natural language processing technology or from information text provided by a patient through an information extracting technology.

4. The intelligent triage method according to claim 3, wherein the step of obtaining the identification knowledge information comprises:

   retrieving key words or key phrases related document contents from the medical literature based on the key words or the key phrases in the candidate factor information by a content retriever;
   finding and mining disease-related knowledge from the retrieved document contents using the natural language processing technology by a knowledge extractor.

5. The intelligent triage method according to claim 4, wherein the step of retrieving key words or key phrases related document contents from the medical literature comprises:

performing participle and named-entity identification to the medical literature using the natural language processing technology and setting up inverted indexes by the content retriever; and,
retrieving a document containing all the key words or key phrases from the inverted indexes based on the key words or the key phrases in the candidate factor information by the content retriever.

6. The intelligent triage method according to claim 5, wherein the step of finding and mining disease-related knowledge from the retrieved document contents using the natural language processing technology comprises: determining segments in the document where the key words or the key phrases locate, and extracting symptom and sign related candidate diseases through sematic relation, by the knowledge extractor.

7. The intelligent triage method according to claim 1, wherein the step of matching the identification knowledge information with the candidate factor information comprises:

if only one of the candidate diseases is outputted, determining the disease and a corresponding affiliated department based on the candidate factor; and
if a plurality of the candidate diseases are outputted, determining the affiliated department based on prevalence of diseases with the same symptoms and signs in a specified period of time or a specified region; or, further selecting information having maximum discrimination between different diseases with crossed symptoms and signs as an extension question, to further obtain patient information and extract new candidate factor information so as to further perform matching.

8. The intelligent triage method according to claim 7, wherein the discrimination between different diseases with crossed symptoms and signs is determined through information gains, the larger an information gain is, the larger the discrimination will be, the smaller an information gain is, the smaller the discrimination will be, the calculation formula of the information gain is:

$$IG(Symptom) = H(Disease) - H(Disease|Symptom)$$

wherein, Symptom represents a symptom, Disease represents a disease, H(.) represents an entropy.

9. An intelligent triage device comprising a candidate factor analyzer, an identification knowledge miner and a matcher wherein:

the candidate factor analyzer is configured to extract disease-related symptoms and signs from patient information as candidate factor information;
the identification knowledge miner is connected with the candidate factor analyzer, and is configured to obtain a plurality of symptom-related candidate diseases and treatment measures from medical literature as identification knowledge information based on the candidate factor information; and
the matcher is connected with the identification knowledge miner, and is configured to match the identification knowledge information with the candidate factor information so as to determine an affiliated department of a disease, and return the determined department as a triage result.

10. The intelligent triage device according to claim 9, wherein the device further comprises a human-computer interaction facility connected with the candidate factor analyzer and the matcher respectively, and the human-computer interaction facility provides a human-computer interaction interface for a patient, and is configured to collect patient information and display the triage result to the patient wherein the patient information is obtained from oral expressions or electronic inputs of the patient, and the triage result is returned to the patient through voice broadcasting or electronic information text display.

11. The intelligent triage device according to claim 9, wherein the candidate factor information includes key words or key phrases and time events of the disease-related symptoms and signs, and the candidate factor analyzer is configured to extract the key words or key phrases and time events from oral expressions provided by a patient through natural language processing technology or from information text provided by a patient through information extracting technology.

12. The intelligent triage device according to claim 11, wherein the identification knowledge miner comprises a content retriever and a knowledge extractor, and wherein:

the content retriever is configured to retrieve key words or key phrases related document contents from the medical literature based on the key words or the key phrases outputted by the candidate factor analyzer; and the knowledge extractor is configured to find and mine disease-related knowledge from the retrieved document contents using the natural language processing technology.

13. The intelligent triage device according to claim 12, wherein the content retriever is configured to:

perform participle and named-entity identification to the medical literature using the natural language processing technology and set up inverted indexes; and retrieve a document containing all the key words or key phrases from the inverted indexes based on the key words or the key phrases outputted by the candidate factor analyzer.

14. The intelligent triage device according to claim 13, wherein the knowledge extractor is configured to determine segments in the document where the key words or the key phrases locate, and extract symptom and sign related candidate diseases through sematic relation.

15. The intelligent triage device according to claim 9, wherein if only one of the candidate diseases is outputted, the matcher is configured to determine the disease and a corresponding affiliated department based on the candidate factor; and
if a plurality of the candidate diseases are outputted, the matcher is configured to determine an affiliated department based on prevalence of diseases with the same symptoms and signs in a specified period of time or a specified region; or, the matcher is configured to further select information having the maximum discrimination between different diseases with crossed symptoms and signs as an extension question, to further obtain patient information and extract new candidate factor information so as to further perform matching.

16. The intelligent triage device according to claim 15, wherein the matcher is configured to determine discrimination between different diseases with crossed symptoms and signs through information gains, the larger an information gain is, the larger the discrimination will be, the smaller an information gain is, the smaller the discrimination will be, the calculation formula of an information gain is:

$$IG(Symptom) = H(Disease) - H(Disease|Symptom)$$

wherein, Symptom represent a symptom, Disease represents a disease, H(.) represents an entropy.

17. An intelligent triage device, comprising:

one or more processors; and
a memory storing computer executable instructions thereon, the computer executable instructions being configured to, when executed by the one or more processors, carry out the method according to any one of claims 1 to 8.

18. A computer readable storage medium containing computer executable instructions thereon, the instructions, when executed by one or more processors, enabling the one or more processors to carry out the method according to any one of claims 1 to 8.

```
┌─────────────────────────────────────────────┐
│      collecting patient information through a   │ ⟋ S1
│       human-computer interaction interface      │
└─────────────────────────────────────────────┘
                      │
                      ▼
┌─────────────────────────────────────────────┐
│  extracting disease-related symptoms and signs from the │ ⟋ S2
│   patient information as candidate factor information by  │
│            a candidate factor analyzer            │
└─────────────────────────────────────────────┘
                      │
                      ▼
┌─────────────────────────────────────────────┐
│  obtaining a plurality of symptom-related candidate diseases │ ⟋ S3
│      and treatment measures from medical literature as      │
│  identification knowledge information based on the candidate │
│  factor information by an identification knowledge miner  │
└─────────────────────────────────────────────┘
                      │
                      ▼
┌─────────────────────────────────────────────┐
│   matching the identification knowledge information with   │ ⟋ S4
│      the candidate factor information by a matcher       │
└─────────────────────────────────────────────┘
                      │
                      ▼
┌─────────────────────────────────────────────┐
│  determining an affiliated department of a disease or  having  │ ⟋ S5
│  extracted and matched the patient information, and returning │
│   the affiliated department of the disease as a triage result  │
└─────────────────────────────────────────────┘
```

Fig. 1

Fig. 2

Fig. 3

| **INTERNATIONAL SEARCH REPORT** | International application No. |
| | PCT/CN2017/116370 |

### A. CLASSIFICATION OF SUBJECT MATTER

G06F 19/00 (2018.01) i

According to International Patent Classification (IPC) or to both national classification and IPC

### B. FIELDS SEARCHED

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNKI, CNPAT, WPI, EPODOC: 智能, 分诊, 关键词, 症状, 匹配, 疾病, 语音, 分词, 索引, 抽取, disease, match, intelligent, symptom, keyword, index, voice, guide, extract

### C. DOCUMENTS CONSIDERED TO BE RELEVANT

| Category* | Citation of document, with indication, where appropriate, of the relevant passages | Relevant to claim No. |
| --- | --- | --- |
| X | CN 102184315 A (INSTITUTE OF MEDICAL INFORMATION, CHINESE ACADEMY OF MEDICAL SCIENCES), 14 September 2011 (14.09.2011), description, paragraphs [0039]-[0064] | 1-18 |
| X | CN 103164616 A (HANGZHOU ZHUOJIAN INFORMATION TECHNOLOGY CO., LTD.), 19 June 2013 (19.06.2013), description, paragraphs [0054]-[0083] and [0130]-[0133] | 1-18 |
| A | CN 106295186 A (INSTITUTE OF COMPUTING TECHNOLOGY, CHINESE ACADEMY OF SCIENCES), 04 January 2017 (04.01.2017), entire document | 1-18 |
| A | CN 103870673 A (BEIJING TIANPENG HENGYU SCIENCE & TECHNOLOGY DEVELOPMENT CO., LTD.), 18 June 2014 (18.06.2014), entire document | 1-18 |

☐ Further documents are listed in the continuation of Box C.     ☒ See patent family annex.

| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| 02 March 2018 | 14 March 2018 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>ZOU, Yuting<br><br>Telephone No. (86-10) 53961440 |

Form PCT/ISA/210 (second sheet) (July 2009)

## INTERNATIONAL SEARCH REPORT
### Information on patent family members

| | International application No. |
|---|---|
| | PCT/CN2017/116370 |

| Patent Documents referred in the Report | Publication Date | Patent Family | Publication Date |
|---|---|---|---|
| CN 102184315 A | 14 September 2011 | None | |
| CN 103164616 A | 19 June 2013 | None | |
| CN 106295186 A | 04 January 2017 | None | |
| CN 103870673 A | 18 June 2014 | None | |

Form PCT/ISA/210 (patent family annex) (July 2009)

**REFERENCES CITED IN THE DESCRIPTION**

**Patent documents cited in the description**

- CN 201710333821